# EUROPÄISCHE PATENTSCHRIFT

(11) **EP 1 402 055 B1**
(45) Veröffentlichungstag und Bekanntmachung des Hinweises auf die Patenterteilung: **24.01.2007**
(21) Anmeldenummer: 02773994.5
(22) Anmeldetag: 29.05.2002
(51) Int. Cl.: C12Q 1/00, C12N 11/02

(54) **VERFAHREN ZUM ERZEUGEN EINER SCHICHT FUNKTIONELLER MOLEKÜLE**
METHOD FOR PRODUCING A LAYER OF FUNCTIONAL MOLECULES
PROCEDE POUR PRODUIRE UNE COUCHE DE MOLECULES FONCTIONNELLES

(30) Priorität: 29.05.2001 AT 8432001
(43) Veröffentlichungstag der Anmeldung: 31.03.2004
(73) Patentinhaber: NANO-S Biotechnologie GmbH, 1180 Wien (AT)
(72) Erfinder: SLEYTR, Uwe, B., 1220 Wien (AT); SARA, Margit, 2230 Gänserdorf (AT); KAUTEK, Wolfgang, 1190 Wien (AT)
(74) Vertreter: Patentanwaltskanzlei Matschnig
(86) Internationale Anmeldenummer: PCT/AT2002/000165
(87) Internationale Veröffentlichungsnummer: WO 2002/097118

(56) Entgegenhaltungen:
- EP-A- 0 189 019
- EP-A- 0 463 859
- WO-A-01/81425
- US-A- 4 752 395
- US-A- 4 886 604
- SLEYTR UWE B ET AL: "Two-dimensional protein crystals (S-layers): Fundamentals and applications." JOURNAL OF CELLULAR BIOCHEMISTRY, Bd. 56, Nr. 2, 1994, Seiten 171-176, XP001097307 ISSN: 0730-2312
- PUM D ET AL: "The application of bacterial S-layers in molecular nanotechnology" TRENDS IN BIOTECHNOLOGY, ELSEVIER, AMSTERDAM, NL, Bd. 17, Nr. 1, Januar 1999 (1999-01), Seiten 8-12, XP004155527 ISSN: 0167-7799
- JAP BK ET AL: "2D crystallization: from art to science" ULTRAMICROSCOPY, AMSTERDAM, NL, Bd. 46, 1992, Seiten 45-84, XP002096893 ISSN: 0304-3991
- KUEPCUE S ET AL: "THE CRYSTALLINE CELL SURFACE LAYER FROM THERMOANAEROBACTER THERMOHYDROSULFURICUS L111-69 AS AN IMMOBILIZATION MATRIX: INFLUENCE OF THE MORPHOLOGICAL PROPERTIES AND THE PORE SIZE OF THE MATRIX ON THE LOSS OF ACTIVITY OF COVALENTLY BOUND ENZYMES" BIOTECHNOLOGY AND APPLIED BIOCHEMISTRY, ACADEMIC PRESS, US, Bd. 21, Nr. PART 3, 1. Juni 1995 (1995-06-01), Seiten 275-286, XP000646028 ISSN: 0885-4513
- SLEYTR U B ET AL: "Application potential of 2D protein crystals (S-layers)." ANNALS OF THE NEW YORK ACADEMY OF SCIENCES. UNITED STATES 30 NOV 1994, Bd. 745, 30. November 1994 (1994-11-30), Seiten 261-269, XP001099023 ISSN: 0077-8923

## Beschreibung

Die vorliegenden Erfindung bezieht sich auf ein Verfahren zum Erzeugen einer Schicht funktioneller Moleküle auf einer Trägeroberfläche unter Verwendung von Zelloberflächenschicht-Proteinen (S-Schichten-Proteinen) als Träger der funktionellen Moleküle, bei welchem eine S-Schicht-Proteine in Form von Monomeren oder Oligomeren enthaltende Lösung mit der Trägeroberfläche in Kontakt gebracht wird, eine Lage von S-Schicht-Proteinen auf der Trägeroberfläche abgeschieden wird und in der so gebildeten Lage eine zweidimensionale kristalline Struktur ausgebildet wird.

Die Herstellung von rekristallisierten S-Schichten aus proteinhältigen kristallinen Zelloberflächenschichten (bakterielle Zelloberflächenschichten) auf festen oder flüssigen Substraten ist beispielsweise in der EP 0 154 620 B1 (= US 4,752,395) beschrieben, die insbesondere die Herstellung von mehrlagigen S-Schichten aus Prokaryonten und deren Verwendung als Ultrafiltrationsmembranen betrifft.

S-Schichten bestehen aus Proteinen, die in Prokaryonten als Zellwandbausteine vorkommen, wobei die Aminosäuresequenz eines S-Schicht-Proteins im allgemeinen Art- oder Stammspezifisch ist; die S-Schicht-Proteine sind in der Regel einfache Proteine oder Glykoproteine. Derzeit sind mehrere hundert Bakterienstämme bekannt, deren Zelloberflächen kristalline Strukturen aufweisen. Die Gitterkonstanten dieser Strukturen liegen im Bereich von ca. 3 bis 35 nm, und die S-Schichten sind als kristalline monomolekulare Schichten zumeist 3 bis 30 nm dick.

Isolierte - von der Ursprungszelle abgelöste - S-Schicht-Proteine zeigen die Fähigkeit, aus einer Lösung zu monomolekularen Kristallgittern (S-Schichten) zusammen zu treten, die den Kristallverbänden in der Zellwand einer intakten Zelle entsprechen. Generell ist eine vollständige Desintegration von S-Schichten in konzentrierten Lösungen chaotroper Agenzien sowie durch Senkung oder Erhöhung des pH-Wertes erzielbar. Bei dem Wiederzusammentreten der S-Schicht-Proteine ("Rekristallisation") können sich flache Schichten, Röhrenstrukturen wie z.B. Zylinder, sowie geschlossene Strukturen (Vesikel) ausbilden - sogenannte Self-Assembly-Produkte -; und zwar in Abhängigkeit von den intrinsischen Eigenschaften der S-Schicht-Proteine und den Bedingungen bei der S-Schicht-Bildung, wie z.B. pH-Wert, Ionenstärke und Ionenzusammensetzung der Lösung; neben Monoschichten können sich auch mehrlagige S-Schichten ausbilden. Die Ausbildung der S-Schichten erfolgt durch Selbstorganisation ('self-assembly'), da sämtliche Information zur Ausbildung der S-Schicht-Struktur in den individuellen Monomeren enthalten ist. Die S-Schichten bilden sich bevorzugt an Grenzschichten aus, z.B. an der Luft-Wasser-Grenze, auf Lipid-Filmen oder auf einer festen Substratoberfläche wie jene eines Silizium-Wafers, einer Elektrode oder eines synthetischen Polymers. Durch die spezifischen Oberflächeneigenschaften der S-Schicht-Proteineinheiten, wie z.B. Ladungsverteilung, Hydrophobie, spezifische Wechselwirkungen mit Komponenten der Grenzschicht, kommt es zu einer definierten Orientierung der S-Schicht-Einheiten (Monomere, Oligomere oder kleine Kristallite) beim Anlagern an die Grenzschicht und Einbau ins S-Schicht-Gitter.

S-Schichten stellen hochporöse Membranen dar, wobei die Poren als Teil des S-Schicht-Gitters einheitliche Größe und Gestalt im Bereich von ca. 2-6 nm aufweisen; die Porosität kann bis zu 70% der Fläche ausmachen. Es ist bekannt, S-Schicht-Gitter wegen der hohen Dichte sowie definierten Position und Orientierung von funktionellen Gruppen, die an der Oberfläche der S-Schicht-Einheiten angeordnet sind, zur Immobilisierung verschiedener (Makro)Moleküle zu nutzen. Um kovalente Bindungen zwischen den S-Schicht-Subeinheiten zu erzeugen und die Stabilitätseigenschaften zu verbessern, wurden S-Schicht-Gitter mit homobifunktionellen, Aminogruppen-spezifischen Vernetzern verschiedener Länge (z.B. Glutaraldehyd) quervernetzt. Viele Enzyme, die auf aktivierten Endgruppen der S-Schicht-Proteine immobilisiert wurden, bildeten eine Monoschicht dicht angeordneter Moleküle auf der äußeren Oberfläche des S-Schicht-Gitters aus. Die sehr spezifischen morphologischen und biophysikalischen Eigenschaften von S-Schicht-Proteinen, besonders ihre Fähigkeit, sich zu monomolekularen kristallinen Gittern zu organisieren, die an ihrer Oberfläche eine räumlich definierte Anordnung funktioneller Gruppen sowie von Poren gleicher Größe und Morphologie aufweisen, haben zu einem breiten Spektrum an Anwendungen in der Biotechnologie, Nanotechnologie und Biomimetik geführt.

Für nähere Details zu S-Schichten sei auf den Artikel 'Crystalline Bacterial Cell Surface Layers (S-Layers): A Versatile Self-Assembly System' von U.B. Sleytr *et al.,* Kapitel 5 aus "Supramolecular Polymers", Ed. A. Ciferri, Marcel Dekker Inc., New York 2000 (ISBN 0-8247-0252-2), verwiesen.

Eine Verwendung von S-Schichten zur Herstellung von Sensorsystemen beschreiben A. Neubauer *et al.* in Sensors and Actuators B 40 (1997) 231-236. Dieser Artikel behandelt die Abscheidung einer Goldschicht auf eine S-Schicht mittels des sogenannten PLD-Verfahrens ('pulse laser deposition', Abscheidung unter Verwendung eines gepulsten Lasers unter Hochvakuumbedingungen), wobei auf die S-Schicht zuvor Enzyme, wie z.B. Glukose-Oxidase, immobilisiert worden war. Weitere Verfahren zur Abscheidung von Metallschichten auf S-Schichten sind unter anderem von D. Pum *et al.* in Ber. Bunsenges. Phys. Chem. 101 (1997) 1686-1689, und A. Neubauer *et al.* in PTB-Berichte F-34 (1998), S. 75-81 behandelt.

Letzterer Artikel diskutiert auch die elektrochemische Abscheidung einer Metallschicht (z.B. Gold) auf eine S-Schicht, allerdings mit wenig zufriederisbellendem Erfolg, da die abgeschiedene Metallschicht eine Komstruktur aufweist, die der Struktur der darunter liegenden S-Schicht nicht korrespondiert.

Die Bildung von S-Schichten und verschiedene Verwendungen dieser Schichten sind auch in der EP 0 207100 A1 (= US 4,886,604), in der EP 0 189 019 A1 und in den Artikeln Sleytr *et al.,* J. Cell Biochem. 56 (1994) 171-176, sowie Küpcü *et al.,* Biotechnol. Appl. Biochem. 21 (1995) 275-286, beschrieben. Jap *et al.,* Ultramicroscopy 46 (1992) 45-84, diskutiert Techniken und Prinzipien der 2-dimensionalen Kristallisierung von Membranen und wasserlöslichen Proteinen.

Bei der Ablagerung einer S-Schicht aus einer Lösung, die S-Schicht-Proteine enthält, scheiden sich die S-Schicht-Einheiten gerichtet auf die Substratoberfläche ab. Sie liegen auf der Oberfläche zunächst ungeordnet vor, die Ausbildung der kristallinen Struktur erfolgt erst im Laufe des Abscheidevorgangs, gewöhnlicher Weise von Kristallisationskeimen ausgehend. Insbesondere wenn die Dichte der S-Schicht-Proteine auf der Substratoberflätche noch nicht sehr groß ist, können sich die einzelnen Einheiten auf der Oberfläche umher bewegen, wobei die Beweglichkeit unter anderem von der Art der Oberfläche anhängt. Der Übergang in eine geordnete Struktur erfolgt häufig spontan, wenn S-Schicht-Einheiten in ausreichender Menge auf der Substratoberfläche abgeschieden sind. Auch nach der Ausbildung einer kristallinen S-Schicht kann noch Abscheidung gelöster S-Schicht-Proteine aus der Lösung und Anlagerung an das S-Schicht-Gittar stattfinden.

Bei bekannten Abscheidungsverfahren treten auch schon in der Lösung S-Schicht-Einheiten zu S-Schichten und S-Schicht Self-Assembly Produkten undefinierter Größe zusammen, die in Lösung oder Suspension verbleiben oder sich auf das Substrat abscheiden. Dieser Vorgang steht somit in Konkurrenz zur direkten Abscheidung von S-Schicht-Einheiten auf das Substrat. Dadurch gestaltet sich die Kontrolle des Herstellungsvorgangs einer S-Schicht schwierig - im besonderen wenn zuverlässig eine Monoschicht abgeschieden werden soll. Des weiteren kann es beim Abscheidungsprozess von S-Schicht-Einheiten zur Ausbildung kristalliner Doppel- und Mehrfachschichten kommen. Dieser Vorgang ist für viele Anwendungsfälle sehr störend, da die zweite (aufliegende) S-Schicht zumeist spiegelverkehrt an die erste (auf der Substratoberfläche gebundene) S-Schicht gebunden ist und somit deren funktionelle S-Schicht-Domänen, die für die Anwendung benötigt werden, maskiert.

Außerdem sind die bekannten Verfahren hinsichtlich der langreichweitigen Ordnung der erzeugten S-Schicht-Strukturen verbesserungswürdig. Insbesondere enthält bei den kristallinen Strukturen, die mittels der genannten Verfahren erzeugt werden, die S-Schicht-Struktur unweigerlich eine Vielzahl von Domänen, deren Ausdehnung somit eher klein ist. Um Domänen großer Fläche zu erhalten, ist es wünschenswert die Zahl der Domänen bzw. die Zahl der Keime, an denen die Ausbildung der Kristallstruktur der S-Schicht ihren Ausgangspunkt nimmt, klein zu halten.

Die EP 463 859 A2 beschreibt die Ablagerung einer Biomolekül-Spezies (z.B. eines Proteins wie Glucose-Oxidase) auf einer Biosensor-Elektrode. Dabei wird ausgehend von einer Lösung, in der die Biomolekül-Spezies das gleiche elektrische Ladungsvorzeichen aufweisen, ein konstanter Strom zwischen der Biosensor-Elektrode und einer Gegenelektrode angelegt, so dass zwischen diesen Elektroden die Biomoleküle zur Biosensor-Elektrode wandern und sich dort als Film ansammeln. Diese Druckschrift beruht somit auf einem galvanostatischen Verfahren, bei dem auf eine kristallartige Ordnung der abgeschiedenen Biomoleküle keine Rücksicht genommen wird; die abgeschiedenen Schichten sind zudem mit ca. 1 µm oder mehr verhältnismäßig dick - im Gegensatz zu den gegenständlichen S-Schichten, bei denen eine oder einige wenige (kristalline) Monolagen zu je 4 bis 15 nm Dicke erzeugt werden.

Es ist daher Aufgabe der vorliegenden Erfindung, die Bildung einer S-Schicht und die Formierung der S-Schicht-Kristallstruktur in gegenüber den bekannten Verfahren verbesserter Weise steuern zu können.

Diese Aufgabe wird von einem Verfahren der eingangs genannten Art gelöst bei welchem zum Abscheiden der S-Schicht-Proteine und zum Ausbilden der kristallinen Struktur verschiedene elektrochemische Bedingungen an der elektrochemischen Grenzschicht auf der Trägeroberfläche eingestellt werden, wobei nach der Abscheidung der S-Schicht-Proteine die Kristallisation und Fixierung der S-Schicht-Proteine durch Einstellung des elektrischen Potenzials des Substrats gegenüber einer Referenzelektrode elektrochemisch kontrolliert werden.

Nach der Erfindung wird die Ausbildung der S-Schicht auf der Substratoberfläche durch elektrochemische Manipulation des Lösung-Substrat-Systems kontrolliert Das Einstellen des elektrischen Potenzials geschieht zweckmäßiger Weise potentiostatisch; der hierbei allfällig auftretende Strom über die Elektrode ist hierbei von untergeordneter Bedeutung. Durch zeitliche Variation der elektrochemischen Parameter und/oder durch Einstellen unterschiedlicher Bedingungen in der Lösung und auf der Substratoberfläche mittels elektrochemischer Methoden gelingt eine verbesserte Kontrolle der Ausbildung der S-Schicht, und die Auswahl eines gewünschten Prozessweges der S-Schicht-Bildung ist möglich, was beispielsweise die Herstellung von S-Schicht-Monoschichten oder Schichten mit einer gewünschten Anzahl oder Abfolge von S-Schichten deutlich erleichtert. Des weiteren kann eine schnellere Belegung der Substratoberfläche und Ausbildung monomolekularer, kristalliner S-Schichten insbesondere auf festen Trägem und eine zuverlässige orientierte Ablagerung der S-Schicht-Proteine erzielt werden.

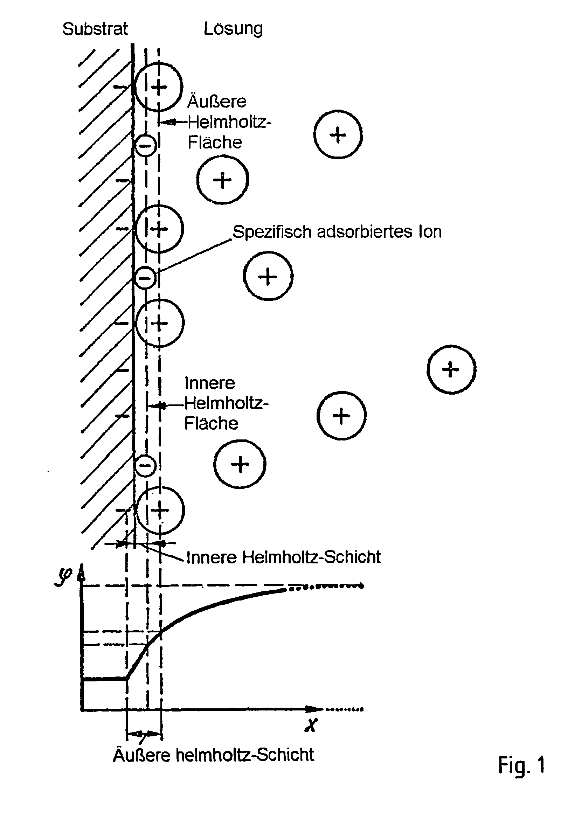

Durch die erfindungsgemäßen elektrochemischen Maßnahmen gelingt die Kontrolle der elektrostatischen Aufladung und der Zusammensetzung der elektrochemischen Doppelschicht auf der Substratoberfläche (sogenannte Helmholtzschicht, Fig.1), und zwar im besonderen der extrinsischen chemischen Zusammensetzung der äußeren Helmholtzschicht, welche aus elektrostatisch gebundenen Ionen besteht, sowie der inneren Helmholtzschicht, die durch chemisch gebundene geladene Teilchen gebildet wird. Des weiteren ermöglicht die Erfindung eine Migrationskontrolle der Adsorption und Desorption durch die Kontrolle des diffusen Teils der elektrochemischen Doppelschicht, der sogenannten diffusen Schicht (oberhalb der Helmholtzschicht). In diesen Schichten stellen sich direkt über der Substratoberfläche chemische Bedingungen ein, die von den chemischen Bedingungen in der Lösung verschieden sind und für die Zwecke der Abscheidung und/oder Kristallisation der S-Schicht-Proteine eingestellt werden. So können z.B. in der Schicht unmittelbar über der Substratoberfläche Feldstärken im Bereich von 10⁵ V/cm auftreten. Diese Feldstärken stellen einen wesentlichen Aspekt bei der Anlagerung der S-Schicht-Proteine an die Substratoberfläche aus der Lösung dar.

Besonders nennenswerte Vorteile der Erfindung sind:
(1) Möglichkeit der verbesserten molekularen Reinigung, Erneuerung und reproduzierbaren Formierung des Substrats noch vor der Abscheidung der S-Schicht, da dies ebenfalls mit elektrochemischen Methoden *in situ* durchgeführt werden kann;
(2) Erreichbarkeit einer hohen Oberflächenkonzentration von S-Schicht-Subeinheiten (Monomeren), S-Schicht-Oligomeren oder -Kristalliten (z.B. als Nukleationskeime), wobei vor der Ausbildung der Kristallstruktur auch ein Konzentrationsüberschuss ('Surface Excess') erreichbar ist;
(3) verbesserte molekulare Orientierung, z.B. hinsichtlich der Außen- und Innenseite der S-Schicht-Subeinheiten und der Nukleationskeime;
(4) kontrollierte Durchführbarkeit einer Protonierung der S-Schicht-Subeinheiten und/oder der Substratoberfläche, wodurch die Natur der Bindung an die Substratoberfläche (Fixierung) bestimmbar ist;
(5) Durchführbarkeit einer Koadsorption (physikalische und/oder chemische Adsorption) von kristallisationsfördemden Substanzen, z.B. ein- oder zweiwertigen Ionen, aus der Lösung oder aus dem Substrat an die S-Schicht;
(6) Durchführbarkeit einer Koadsorption von Reaktanden, z.B. Fixierungsförderern;
(7) Durchführbarkeit einer elektrochemischen Konservierung (d.h. Strukturstabilisierung) und/oder elektrochemischen Modifikation der S-Schicht-Subeinheiten oder S-Schicht-Kristalle bzw. -Nukleationskeime;
(8) vereinfachte Qualitätskontrolle;
(9) Durchführbarkeit getrennter Verfahrensschritte zur Adsorption von S-Schicht-Proteinen und Rekristallisiation;
(10) Durchführbarkeit kontinuierlicher Verfahren, wobei die Substratreinigung, die Adsorption von S-Schicht-Proteinen und die Rekristallisation in getrennten Schritten, gegebenenfalls räumlich getrennt (z.B. verschiedene Gefäße), erfolgen kann.

Als Substrate kommen grundsätzlich beliebig geformte und dimensionierte kondensierte Phase in Betracht, wie z.B. Platten, poröse Körper oder disperse Phasen (z.B. Suspensionen).

Durch elektrochemische Kontrolle der Substratoberfläche können beim Ausbilden des Kristallgitters Bedingungen eingestellt werden, die optimal für die Kristallisation der gebundenen S-Schicht-Subeinheiten sind. Angestrebt werden insbesondere Bedingungen, unter denen sich die gebundenen S-Schicht-Subeinheiten möglichst ungehindert, insbesondere durch laterale Diffusion, zu großflächigen, kristallinen S-Schicht-Domänen umordnen können. Da S-Schicht-Proteingitter auf der Außen- und Innenseite zumeist eine unterschiedliche Nettoladung aufweisen, erfolgt auch die Bindung von S-Schicht-Proteinen auf geladenen Oberflächen in streng definierter Orientierung. Somit kann durch eine kontrollierte Einstellung der Oberflächenladung eines festen Trägers eine gerichtete Adsorption von gelösten S-Schicht-Proteinen (und Nukleationskeimen, d.h. S-Schicht-Kristalliten) erreicht werden. Da die Oberflächenladung von S-Schicht-Proteinen zudem vom pH-Wert der Proteinlösung abhängt, ist der Adsorptionsvorgang (Orientierung der S-Schicht-Proteine bei der Adsorption) sowie die Stärke der Bindung der S-Schicht-Proteine an der Oberfläche des festen Trägers durch Variation der Milieubedingungen beeinflussbar und optimierbar. Eine ladungsabhängige Adsorption und Kristallisation von S-Schicht-Proteinen kann auch zu einer gezielten Abscheidung an strukturierten Oberflächen (z.B. Halbleitermaterialien, Leiterplatten, leitfähigen Polymeren, kompositen Materialien) genutzt werden.

Die Erfindung gestattet zudem die Ausbildung mehrerer S-Schichten auf die Substratoberfläche in definierter Reihenfolge durch entsprechende Wiederholung der jeweils zur Abscheidung/Kristallisation erforderlichen Bedingungen.

In einer bevorzugten Weiterbildung der Erfindung erfolgen das Abscheiden der S-Schicht-Proteine und das Ausbilden der kristallinen Struktur in der abgeschiedenen Proteinschicht voneinander zeitlich getrennt und unter verschiedenen elektrochemischen Bedingungen der Lösung und/ oder des Substrats. In diesem Fall liegen die S-Schicht-Subeinheiten zwischen dem Abscheiden und der Kristallisation ungeordnet auf der Substratoberfläche vor und können auf dieser lateral wandern. Die optimalen Milieubedingungen für die Adsorption von S-Schicht-Monomeren/Oligomeren einerseits und für die Kristallisation der an der Oberfläche gebundenen Proteine andererseits sind im allgemeinen verschieden; sie können in zeitlicher Abfolge eingestellt werden, beispielsweise durch kontinuierliche Veränderung der betreffenden Parameter.

Hierbei kann die Änderung der elektrochemischen Bedingungen vorteilhafter Weise darin bestehen, dass zwischen Abscheiden und Kristallisation das elektrochemische Potenzial der Trägeroberfläche gegenüber der Lösung geändert wird. Außerdem kann zur Änderung der elektrochemischen Bedingungen zwischen Abscheiden und Kristallisation zumindest ein chemischer Parameter der Lösung variiert werden. Als chemische Parameter kommen im besonderen die Konzentration bzw. Ionenstärke des Leitelektrolyts, der pH-Wert, die Sauerstoff-Konzentration oder allgemeiner das Potenzial für Elektronentransfer-Reaktionen sowie die Ionenkonzentration bzw. Konzentration gelöster Stoffe in Frage. Darüber hinaus kann der Prozess auch über die Konzentration anderer Ionen der Lösung oder die Temperatur beeinflusst werden. Ebenso kann zur Änderung der elektrochemischen Bedingungen, anstelle oder in Kombination zu den vorgenannten Parametern, zwischen Abscheiden und Kristallisation zumindest ein elektrochemischer Parameter des Substrats variiert werden, z.B. dessen Ladung, die Bedeckung mit Ionen (beispielsweise Hydroxyl-Ionen) oder chemische Modifikation (Morphologie auf mesoskopischer und/oder atomarer Dimension, wie Rauheit).

Das Potenzial der Lösung kann vorteilhafter Weise stromfrei mittels einer Referenzelektrode gemessen werden, die zusätzlich zu der Elektrode der Trägeroberfläche und einer Gegenelektrode vorgesehen ist. Zweckmäßiger Weise wird die Referenzelektrode in geringem Abstand zu der Oberfläche jener Elektrode angeordnet, für die primär die Potenzialdifferenz zur Lösung bestimmt werden soll; die Potenzialdifferenz der Gegenelektrode ergibt sich dann mittelbar durch die Stromregelung eines Potentiostaten, damit Ist- und Soll-Spannung an der Elektrode der Trägeroberfläche übereinstimmen.

In einer weiteren vorteilhaften Variante der Erfindung wird die elektrische Nettoladung der S-Schicht-Proteine diesen elektrostatisch aufgeprägt. Beispielsweise können durch Veränderung der Oberflächenladung des Trägers nach der Proteinadsorption optimale Bedingungen für die Kristallisation eingestellt werden. Zudem ist es möglich, nach der Ausbildung kristalliner S-Schichten durch die Anlegung von Spannungen an Oberflächen auch weitere geladene Moleküle oder Metall-Cluster an der Oberfläche der S-Schicht-Gitter oder in den Poren zu binden bzw. abzuscheiden.

Darüber hinaus ist es günstig, wenn bei dem Abscheiden der S-Schicht-Proteine und/oder dem Ausbilden der kristallinen Struktur eine Konformationsänderung der Proteine eintritt, insbesondere eine Denaturierung bzw. Renaturierung. Dies erleichtert zusätzlich die Steuerung der Ausbildung der S-Schicht.

Das Abscheiden der S-Schicht-Proteine und/oder das Ausbilden der kristallinen Struktur kann durch einen zeitlich variierten Potenzialverlauf, z.B. Pulse oder Rampen, gesteuert werden.

Die Protein-Adsorption und -Kristallisation können auch in verschiedenen Gefäßen oder Gefäßkompartimenten unter den jeweils für die Prozessschritte einstellbaren Bedingungen stattfinden, wobei in den Gefäßen verschiedene Lösungen enthalten sind, die sich z.B. hinsichtlich der gelösten Substanzen unter Beibehaltung der Leitsubstanz und/oder des Lösungsmittels unterscheiden; auch kann dies in einem Gefäß erfolgen, z.B. durch einen kontinuierlichen oder diskontinuierlichen Wechsel der verwendeten Lösung, wie Ablassen der ersten und Einfüllen einer zweiten Lösung. Dem gemäß wird das Abscheiden der S-Schicht-Proteine auf das Substrat in einer ersten Lösung und das Ausbilden der kristallinen Struktur in einer zweiten Lösung durchgeführt. Hierbei ist es günstig, wenn dem Substrat vor dem Eintauchen in die erste Lösung eine Nettoladung elektrostatisch oder elektrophoretisch aufgeprägt wird, welche bei dem Durchgang durch die Lösungen erhalten bleibt; ebenso kann dem Substrat in der ersten Lösung elektrochemisch eine Nettoladung aufgeprägt werden, welche bei dem Wechsel der Lösungen erhalten bleibt. Zusätzlich kann der Wechsel der Lösungen durch Transport der Trägeroberfläche von einem ersten Lösungsbad, in dem das Abscheiden erfolgt, zu einem zweiten Bad für die Kristallisation geschehen (sogenannte Emersion). Dies entspricht einem Batch-Verfahren. In einer Variante hierzu kann der Transport in einem kontinuierlichen Transportprozess erfolgen, in dem das - z.B. band- oder faserförmige - Substrat nach Art eines Fließband hintereinander durch verschiedene Bäder hindurchgeführt wird, wobei sich zur gleichen Zeit verschiedene Teile desselben Substrats in jeweils einem anderen Bad befinden können.

Die Immobilisierung der funktionellen Moleküle an der S-Schicht kann beispielsweise dadurch erfolgen, dass funktionelle Moleküle an S-Schicht-Proteine noch vor der Abscheidung der S-Schicht-Proteine gebunden werden und sodann die funktionellen Moleküle auf das Substrat zugleich mit dem Abscheiden der S-Schicht-Proteine abgelagert werden. Hierbei ist es zweckmäßig, wenn die funktionellen Moleküle ausschließlich oder zumindest zu einem großen Teil auf einer Seite der S-Schicht-Proteine - z.B. auf jener Seite, die der Innenseite der Zellwand entspricht - gebunden werden und für das Abscheiden elektrochemische Bedingungen gewählt werden, unter denen die S-Schicht-Proteine ein resultierendes Dipolmoment aufweisen, wobei aufgrund der Ausrichtung des Dipolmoments nahe der Trägeroberfläche im infolge der Potenzialdifferenz bestehenden elektrischen Feld besagte Seite der S-Schicht-Proteine von der Trägeroberfläche abgewandt ist.

Alternativ oder in Kombination hierzu kann die Immobilisierung dadurch geschehen, dass nach der Ausbildung der kristallinen Struktur in der S-Schicht-Lage funktionelle Moleküle auf das Substrat an durch die kristalline Struktur definierte Positionen abgelagert werden.

Als funktionelle Moleküle können unter anderem Enzyme, Virusproteine oder andere Proteinfunktionen verwendet werden. Die funktionelle Einheit kann auch dadurch realisiert sein, dass das S-Schicht-Protein als rekombinantes Fusionsprotein eine oder mehrere funktionelle Einheiten aufweist.

Des weiteren können nach der Ausbildung der kristallinen Struktur in der S-Schicht-Lage elektrochemisch Nanopartikel auf das Substrat an durch die kristalline Struktur definierte Positionen abgelagert bzw. abgeschieden werden. Dies schließt auch die Verwendung der S-Schicht als Templat zur Abscheidung einer zweiten Phase ein.

Im folgenden wird die vorliegende Erfindung anhand einiger nicht einschränkender Ausführungsbeispiele mit Bezug auf die beiliegenden Figuren näher erläutert. In den Figuren zeigen:
- Fig.1: ein Schema des Aufbaus der elektrochemischen Doppelschicht (nach Gouy, Chapman und Stern);
- Fig. 2: eine Vorrichtung zur Durchführung eines kontinuierlichen Beschichtungsprozesses für ein Bandsubstrat;
- Fig. 3: eine Anordnung zur elektrostatischen Aufladung des Bandsubstrats der Fig. 2;
- Fig. 4: eine Vorrichtung zur potentiostatischen S-Schicht-Deposition, beruhend auf einer elektrochemischen Suspensionszelle nach B. Kastening *et al.*;
- Fig. 5: den Vorgang der potentiostatisch induzierten Deposition.

Die im folgenden behandelten Beispiele beschreiben Vorgehensweisen, bei denen gemäß der Erfindung eine Schicht funktioneller Moleküle auf einer elektronisch leitenden Trägeroberfläche unter Verwendung von Monomeren, Oligomeren oder Kristalliten von proteinhältigen Zelloberflächenschichten (S-Schicht-Proteinen) elektrochemisch abgeschieden, zur zweidimensionalen Kristallisation gebracht und schließlich elektrochemisch fixiert wird. Ein Substratmaterial, welches elektrische Ladung leiten und/oder akkumulieren kann, wird dabei derart konditioniert, dass (1) Adsorption, (2) Orientierung, (3) Oberflächendiffusion,

**Tabelle 1: Theoretische isoelektrische Punkte (pI) der reifen S-Schicht-Proteine, der N-Termini, sowie der restlichen Sequenzen. Die Zeile GBAN gibt die GenBank-Zugriffsnummer (GenBank Accession Number) wieder.**

| S-Schicht-Protein-Form | **SbsB** | **SbpA** | **SbsC** |
|---|---|---|---|
| GBAN | X 98095 | AF 211170 | AF 055578 |
| reifes Protein | pI 4.95 Aminosäure 32-920 | pI 4.60 Aminosäure 31-1268 | pI 5.40 Aminosäure 31-1099 |
| N-Terminus | pI 8.94 Aminosäure 32-207 | pI 4.49 Aminosäure 31-202 | pI 9.13 Aminosäure 31-270 |
| Restliche Sequenz | pI 4.73 Aminosäure 208-920 | pI 4.63 Aminosäure 203-1268 | pI 4.88 Aminosäure 271-1099 |

### (4) Kristallisation und (5) Fixierung von S-Schicht-Einheiten elektrochemisch kontrolliert ablaufen.

Mit Hilfe einer potentiostatischen elektrochemischen Steuerung des Substratmaterials lassen sich grundsätzlich drei elektrochemische Oberflächentypen einstellen, die den entscheidenden Einfluss auf die oben genannten Prozesse haben. Der erste Bereich ist dadurch gekennzeichnet, dass eine negative Oberflächenladung vorliegt, der zweite dadurch, dass eine positive Nettoladung eingestellt wird. Der dritte Bereich, in dem noch positivere Potenziale erzeugt werden, zeichnet sich dadurch aus, dass die Oberfläche elektrochemisch umgewandelt wird, sodass nunmehr das Substrat nur mehr über eine hydroxidische oder oxidische Oberfläche oder über andere spezifisch adsorbierte Ionenspezies mit Adsorbaten in Wechselwirkung tritt. In den ersten beiden Bereichen wird die Wechselwirkung zu Adsorbaten überwiegend durch elektrostatische Kräfte bestimmt und erst in zweiter Linie durch chemische (z.B. van-der-Waals-Kräfte). Im dritten Bereich überwiegen dagegen chemische (z.B. Wasserstoffbrückenbindungen) und nicht elektrostatische Wechselwirkungen.

### Beispiel 1

Das Schicht-Protein SbsB - z.B. in seiner rekombinant hergestellten Form, die als rSbsB₃₂₋₉₂₀ bezeichnet wird - weist als reifes Protein einen theoretischen isoelektrischen Punkt (pI) von 4.95 auf. Die N-terminale Region (Aminosäuren 32-207), die drei sog. S-Layer Homologe Motive (SLH-Motive) umfasst, hat einen pI von 8.94; für den Rest der Sequenz (Aminosäuren 208-920) wurde ein pI von 4.73 errechnet (siehe Tabelle 1). Versuche mit rekombinant hergestellten, N-terminal verkürzten SbsB-Formen, denen bis zu 3 SLH-Motive fehlten, bestätigten, dass der N-Terminus nicht in den Self-Assembly Vorgang involviert ist.

Zur Herstellung von löslichem S-Schicht-Protein (S-Schicht-Monomere oder Oligomere für die Kristallisation an festen Oberflächen) wurden 0.2 mg S-Schicht Self-Assembly Produkte von rSbsB₃₂₋₉₂₀ (monomolekulare S-Schicht-Proteinkristalle mit einer Größe von 0.1 bis 5 µm) in 1 ml 5 M Guanidinhydrochlorid (GHCl) in 50 mM TRIS-HCl-Puffer (pH 7.2) gelöst, und 24 Stunden gegen Aq. purif. bei 4°C dialysiert. Aufgrund der geringen Konzentration an S-Schicht-Protein wurde die Ausbildung von Self-Assembly Produkten während der Dialyse verhindert. Gleichwohl wurde nach Beendigung der Dialyse bei 40 000 g für einen Zeitraum von 20 Minuten bei 4°C zentrifugiert, der klare Überstand entnommen, der pH-Wert der Lösung mit 0.01 N NaOH auf 6.5 eingestellt, und 400 µl dieser Lösung zur Kristallisation verwendet.

Zunächst wurde in der Elektrolytlösung ohne S-Schicht-Protein ein Reinigungsschritt durchgeführt, in dem durch wiederholtes Zyklisieren des Elektrodenpotenzials zwischen extremen Werten (z. B. an Gold zwischen -1.2 V und +0.8 V gegen eine gesättigte Quecksilbersulfatelektrode) unter Beibehaltung des pH-Werts eventuelle Kontaminatschichten durch den totalen Austausch der Oberflächenatome des Substrates eliminiert wurden.

Für die nachfolgende S-Schicht-Adsorption kann als fester Träger ein elektrisch leitendes Material verwendet werden. Hierfür kommen insbesondere Metalle, z.B. Gold, Halbleiter wie Silizium, leitfähige Polymere oder andere Materialien wie z.B. Graphit in Betracht.

Durch die eingestellte Nettoladung des festen Trägers kann die Orientierung der adsorbierten S-Schicht-Subeinheiten gesteuert werden. In einem ersten Fall wird die Oberfläche (5 mm²) des festen Trägers so konditioniert, dass sie eine negative Nettoladung aufweist. Unter diesen Bedingungen binden die S-Schicht-Subeinheiten als Monoschicht mit der Innenseite, die den N-Terminus umfasst und bei einem pH-Wert von 6.5 positiv geladen ist. In einem zweiten Fall wird die Oberfläche des festen Trägers so konditioniert, dass sie eine positive Nettoladung aufweist, wobei die S-Schicht-Subeinheiten mit der bei einem pH-Wert von 6.5 negativ geladenen Außenseite binden.

Nach Adsorption der S-Schicht-Subeinheiten wurde die S-Schicht-Proteinlösung entfernt und mehrmals mit Aq. purif. gewaschen. Um die Ausbildung des Kristallgitters zu induzieren, wurde eine Lösung von zweiwertigen Kationen (z. B. 5 mM CaCl₂) in Aq. purif. zugesetzt, und 2 Stunden bei 20°C inkubiert. Da der N-terminale Teil (pI 8.94) nicht in den Self-Assembly Prozess involviert ist, ist vor allem die Neutralisation der negativen Ladungen im Bereich der Aminosäuren 208-920 durch die Zugabe von zweiwertigen Kationen wesentlich. Der Nachweis einer kristallinen S-Schicht-Struktur auf der Substratoberfläche wurde im AFM-Scannermikroskop ('Atomic Force Microscope') durchgeführt. Es konnten einkristalline Bereiche mit einer Größe von etwa 1-10 µm nachgewiesen werden, die allerdings unterschiedliche Orientierung zueinander zeigten.

Wird dagegen für die Adsorption das Potenzial in den dritten Bereich der Oxidbildung verschoben, kann die völlig veränderte Substratoberfläche nunmehr andere vertikale chemische Bindungen (z.B. nicht elektrostatische Wechselwirkungen) zur bereits ausgebildeten kristallisierten S-Schicht eingehen.

### Beispiel 2

In diesen Versuchen wurde ein rekombinant hergestelltes rSbsB-Streptavidin-Fusionsprotein verwendet. Die funktionelle Domäne (Streptavidin) ist am C-terminalen Ende des S-Schicht-Proteins fusioniert. Der pI der Sequenz, die zwischen Aminosäure 208-920 von SbsB liegt sowie das heterotetramere Streptavidin umfasst, beträgt 5.60. Das C-terminale SbsB-Streptavidin-Fusionsprotein ist wasserlöslich, und bildet das schräge S-Schicht-Gitter nur an vorkonditionierten Oberflächen aus (z.B. Zellwandsacculi von *Bacillus stearothermophilus* PV72/p2, die Peptidoglykan vom A1γ-Chemotyp und das für den Organismus typische sekundäre Zellwandpolymer enthalten).

Für die Rekristallisation wurde eine Lösung, die 200 µg des Fusionsproteins in 500 µl Aq. purif. enthielt, mit einer 5 mm² großen negativ geladenen festen Oberfläche in Kontakt gebracht. Die Bindung des Fusionsproteins erfolgte hier über die bei einem pH-Wert von 6.0 positiv geladene N-terminale Region. Zur Stabilisierung des S-Schicht-Gitters wurden 500 µl einer Lösung, die 1-Ethyl-3,3'-Dimethylaminopropyl-Carbodiimid (EDC) als Zero-Length-Crosslinker enthielt (1 mg EDC je ml Aq. purif., wobei der pH-Wert der Lösung mit 0.01 M HCl auf 4.75 eingestellt wurde), zugegeben und 60 Minuten bei 20°C inkubiert. EDC kann nur an jenen Stellen vernetzen, wo Amino- und Carboxylgruppen in direkter elektrostatischer Wechselwirkung vorliegen, wie etwa im Falle von benachbarten S-Schicht-Subeinheiten. Nach der Inkubation wurde der Träger einige Male mit Aq. purif. gewaschen. Anschließend wurde die Struktur des S-Schicht-Gitters im AFM-Mikroskop untersucht. Das schräge S-Schicht-Gitter war deutlich zu sehen. Die unterschiedlich orientierten Bereiche hatten eine Größe von etwa 1-10 µm.

### Beispiel 3

Das reife S-Schicht-Protein SbpA (Aminosäuren 31-1268; pI 4.60; vgl. Tabelle 1) kristallisiert in einem quadratischen Gitter. Der N-terminale Teil (Aminosäuren 31-202) des reifen S-Schicht-Proteins umfasst die 3 SLH-Motive und die Bindungsregion für das sekundäre Zellwandpolymer, und weist einen pI von 4.49 auf. Der pI der restlichen Sequenz (Aminosäuren 203-1268) liegt bei 4.63. Der N-terminale Teil ist in den Self-Assembly Vorgang nicht involviert.

Für die Ausbildung von Self-Assembly Produkten mit quadratischem Gitter sind zweiwertige Kationen erforderlich. Eine Lösung, die 200 µg rSbpA je ml Aq. purif. enthielt und einen pH-Wert von 6.0 aufwies, wurde auf eine 5 mm² große positiv geladene Trägeroberfläche gebracht, und 2 Stunden bei 20°C inkubiert. Anschließend wurde die überschüssige S-Schicht-Protein-Lösung weggewaschen. Zur Ausbildung des Kristallgitters wurde eine 10 mM CaCl₂-Lösung auf die Trägeroberfläche gebracht, und 2 Stunden bei 20°C inkubiert. Nach Waschen mit Aq. purif. wurde der Träger im AFM untersucht. Es waren etwa 0.5 µm große Bereiche mit quadratischem Gitter zu erkennen, die sich jedoch hinsichtlich ihrer Oberflächenstrukturen deutlich unterschieden; die beiden unterschiedlich strukturierten Bereiche lagen etwa im Verhältnis 1 : 1 vor. Daraus wurde geschlossen, dass die S-Schicht-Subeinheiten sowohl mit der Innenseite (raue Oberfläche), wie auch mit der Außenseite (glatte Oberfläche) adsorbieren konnten. Dieses Beispiel zeigt, dass eine einheitlich orientierte Abscheidung der S-Schicht-Subeinheiten auf ein geladenes Substrat nur dann erfolgt, wenn S-Schicht-Subeinheiten auf ihrer inneren und äußeren Oberfläche unterschiedlich geladene Domänen aufweisen (vgl. Beispiele 2 und 3).

### Beispiel 4

Es sollte das rekombinant hergestellte Fusionsprotein rSbsC-Bet v 1 auf festen Oberflächen kristallisiert werden. In dem rSbsC-Bet v 1 ist am C-terminalen Ende einer verkürzten SbsC-Form, die Aminosäuren 31-920 umfasst und einen pI von 6.09 aufweist, die Sequenz des Haupt-Birkenpollen-Allergens Bet v 1 (161 Aminosäuren) über 2 Glycinreste als Linker fusioniert. Das reife SbsC (Aminosäuren 31-1099) weist einen pI von 5.40 auf. Der pI der N-terminalen Region (Aminosäuren 31-270) liegt bei 9.13; jener der restlichen Sequenz (Aminosäure 271-1099) beträgt 4.88 (siehe Tabelle 1). Der N-terminale Teil ist in den Self-Assembly Vorgang nicht involviert.

Zur Kristallisation an einer festen Oberfläche mit negativer Nettoladung wurde eine Lösung von 250 µg rSbsC-Bet v 1-Fusionsprotein je ml Aq. purif. mit einem pH-Wert von 6.0 aufgebracht. Auf diese Weise erfolgte die Adsorption des Fusionsproteins über den positiv geladenen N-Terminus. Die am C-terminalen Ende angehängte Bet v 1-Sequenz blieb demnach nach außen exponiert. Nach einer zweistündigen Inkubation bei 20°C wurde durch Zugabe von 0.01 M HCl ein pH-Wert von 6.09 eingestellt, sodass die negative Nettoladung des C-terminalen S-Schicht-Protein-Anteiles verloren ging, und die Subeinheiten "komprimiert" wurden, d.h. infolge des Verlustes der Nettoladung eine gegenseitige Annäherung erfolgte. Durch diesen Vorgang wurde die regelmäßige Gitterstruktur ausgebildet.

### Beispiel 5

Dieses Beispiel illustriert die kontinuierliche Beschichtung eines Substrats in Form eines Bandmaterials mit S-Schichten.

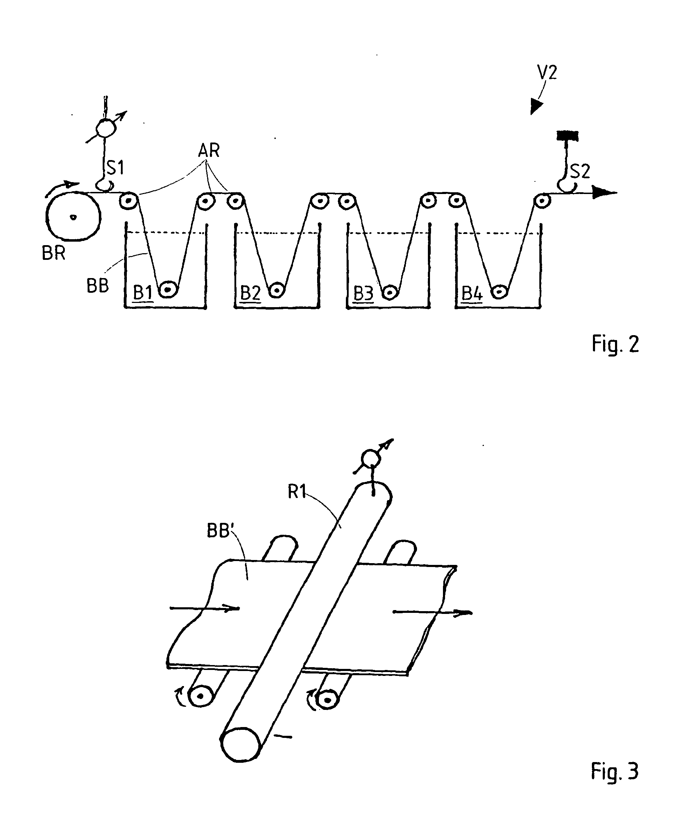

Beispielsweise kann dies mit einer Vorrichtung V2 wie in Fig. 2 gezeigt erfolgen. Das zu beschichtende Band BB, z.B. bestehend aus einem Kunststoff-Trägermaterial, das auf jener Seite, auf der die S-Schicht abgelagert werden soll, metallbeschichtet ist, wird von einer Rolle BR abgespult und über geeignete Ablenkwalzen oder -rollen AR durch die Behälter B1,B2,B3,B4 geführt. Die Zahl der Bäder ist hier natürlich nur beispielsweise gegeben und kann dem gewünschten Prozess angepasst werden, und zwar erhöht oder verringert. Das leitfähige Bandmaterial wird nach bekannter Art über Schleifkontakte S1,S2 aufgeladen bzw. nach der Bäderfolge wieder entladen (Erdung).

In dem hier betrachteten Beispiel sind die einzelnen Behälter wie folgt gefüllt:
- Behälter B1:: Aq. purif. zum Waschen und Reinigen der Folie;
- Behälter B2:: Lösung mit S-Schicht-Protein (rSbsC; 50 µg/ml in Aq. purif. bei pH 5.7) zur Beladung der Folie mit S-Schicht-Protein;
- Behälter B3:: Lösung (20 mM CaCl₂-Lösung in Aq. purif.) zur Kristallisation des adsorbierten S-Schicht-Proteins;
- Behälter B4:: Vernetzerlösung (0.5% Glutaraldehyd in 0.2 M Na-Cacodylat-Puffer, pH 7.0), in der die chemische Vernetzung der kristallinen Schicht zur weiteren Stabilisierung erfolgt.

Die Verweil- bzw. Durchlaufdauer des Bandes in jeweils einem Behälter liegt zwischen 20 und 120 Minuten. Nach der Beschichtung und chemischen Vernetzung können noch weitere (nicht gezeigte) schritte, wie ein Waschschritt und ein Trocknungsschritt durchgeführt werden.

Selbstverständlich kann anstelle der Bäderfolge B1-B4 auch eine andere Bäderfolge verwendet werden, z.B. einem der Beispiele 1 bis 4 entsprechend.

In einer Variante zu diesem Beispiel kann auch eine nicht-leitendes Material, wie z.B. eine Polymerfolie, beschichtet werden. In diesem Fall erfolgt die Aufladung des Bandmaterials nicht über Schleifkontakte wie in Fig. 2, sondern elektrostatisch. Bezugnehmend auf Fig. 3 wird hierzu die Folie BB' in direktem Kontakt oder in geringem Abstand an einer geladenen Walze oder Stab R1 vorbeigeführt und auf diese Weise elektrostatisch aufgeladen. Hierzu wird der Stab R1 zweckmäßigerweise auf eine Hochspannung von beispielsweise 1000 V gelegt. Dieser Stab R1 würde in der Vorrichtung V2 der Fig. 2 anstelle des Schleifkontakts S1 montiert werden.

### Beispiel 6

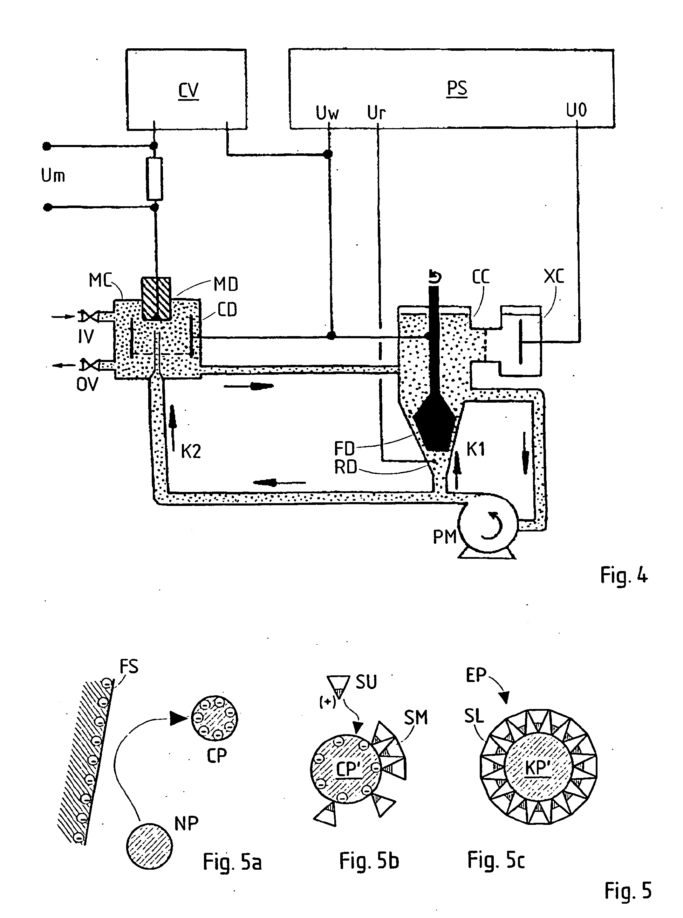

Dieses Beispiel behandelt die Kristallisation eines S-Schicht-Proteins durch ein elektrophoretisches Verfahren, beispielsweise in einer elektrochemischen Suspensionszelle nach B. Kastening *et al.,* in J. Electroanal. Chem. 265 (1989), S. 77-101. In der von Kastening beschriebenen elektrochemischen Suspensionszelle, die in Fig. 4 schematisch gezeigt ist, ist auf der rechten Seite in einem ersten Behälter CC, der als "Fütterkammer" dient, ein rotierender Teil FD aus Graphit von konischer Gestalt und strukturierter Oberfläche vorhanden. Der rotierende Teil FD wird mit Hilfe eines Potentiostaten PS auf einem konstanten Arbeitspotenzial Uw gegenüber dem Potenzial U0 einer Gegenelektrode XC gehalten. Er dient des weiteren als "Fütterelektrode", die den in der Folge in die Zelle eingebrachten Partikeln ein bestimmtes Potenzial verleihen soll.

Die Diagramme der Fig. 5 veranschaulichen den Vorgang der S-Schicht-Kristallisation auf den Partikeln. An der Oberfläche FS der Fütterelektrode FD wird elektrische Ladung auf die in Suspension befindlichen Partikel NP übertragen, die in dem Behälter CC an der Fütterelektrode FD vorbeigeführt werden - Fig. 5a. In dem hier betrachteten Beispiel übernehmen die so elektrophoretisch behandelten Partikel CP eine negative Ladung von der Fütterelektrode. Im nächsten Schritt - Fig. 5b - lagern sich die S-Schicht-Einheiten SU in einer Orientierung auf das Partikel CP' ab, wobei die Orientierung durch die Verteilung der geladenen Domänen und die auf der Partikeloberfläche befindliche Ladung bestimmt ist. Dies erfolgt im hier betrachteten Beispiel über den N-Terminus von rSbsB, der eine positive Nettoladung aufweist und in Fig. 5 als schraffierte Spitze der S-Schicht-Subeinheiten dargestellt ist. Auf der Oberfläche kann auch schon die Formierung von Oligomeren SM stattfinden. Durch weitere Adsorption wird eine vollständige Bedeckung der Partikel erreicht. Durch Kristallisation der S-Schicht erhält man ein von einer zusammenhängenden S-Schicht SL überzogenes Partikel EP. Als Kern KP' für diese Partikel können verschiedene disperse Materialien wie z.B. Metallpartikel aus Au, Pt oder anderen Metallen, Kohlenstoff in Form von Graphitpartikel oder Aktivkohle, Kunststoff-beschichtete Partikel oder magnetische Partikel verwendet werden.

Für die Aufladung der Partikel wird in der Apparatur der Fig. 4 die Lösung mittels einer Pumpe PM in dem Hauptkreislauf K1 an der Fütterelektrode FD vorbeigeführt. Der Behälter CC weist auch eine Referenzelektrode RD zum stromfreien Messen des Potenzials Ur der Lösung auf. Außerdem ist ein Nebenkreislauf K2 vorgesehen, der über eine Messkammer MC führt, in der über eine Messelektrode MD die Ladung der in der Lösung suspendierten Partikel gemessen werden kann; hierzu wird beispielsweise mittels einer Konstantspannungsquelle CV eine Spannung zwischen der Messelektrode MD und einer auf dem Arbeitspotenzial Uw liegenden Zylinderelektrode CD aufgebaut, und aus dem Strom (als Spannung Um gemessen) kann nach bekannter Art die Ladung der Partikel bestimmt werden. Die Messkammer MC kann auch zum Zu- oder Abführen der verwendeten Lösungen und Zusatzstoffe über die Einlass- und Auslassventile IV,OV verwendet werden.

In die elektrochemische Suspensionszelle werden 25 ml einer Suspension aus 5 g Polystyrol-Latex-Kugeln mit einem Durchmesser von 1 µm in einer Pufferlösung (0.1 M Na-Citrat-Lösung, pH 6.0) eingefüllt. Anstelle der Polystyrol-Latex-Kugeln können auch Polystyrol-Kugeln ohne Latexmantel verwendet werden; beide verwendeten Kugelmaterialien sind Produkte der Banks Lab., in Indiana (USA) . Das Potenzial der Fütterelektrode wird so eingestellt, dass die Kugeln eine negative Oberflächenladung annehmen, z.B. auf -1 V gegenüber der gesättigten Quecksilbersulfat-Elektrode XC. Nach einer Pumpzeit von 20 Minuten werden 25 ml einer S-Schicht-Proteinlösung (rSbsB; 0.25 mg je ml Aq. purif., pH-Wert 6.0) eingespritzt, z.B. in den Behälter MC oder über den Nebenkreis K2. Zur gerichteten Adsorption des S-Schicht-Proteins über den an der Innenseite liegenden und bei einem pH-Wert von 6.0 positiv geladenen N-terminalen Teil wird die Suspension 2 Stunden bei 20°C zirkuliert. Dies geschieht zweckmäßigerweise bei Zirkulation im Nebenkreis K2, wobei der Kreis K1 durch ein (nicht gezeigtes) Ventil in der Zuführleitung des Behälters CC geschlossen wird; dadurch wird eine Abscheidung von S-Schicht-Protein auf die Fütterelektrode vermieden. Anschließend wird die Suspension der Zelle entnommen, 20 Minuten bei 40,000 g bei 20°C zentrifugiert, der klare Überstand verworfen, und das verbleibende Zentrifugat - das sogenannte Pellet - in 20 ml einer 10 mM CaCl₂-Lösung suspendiert. Die Suspension wird in der Folge 4 h bei 20°C leicht gerührt. In Gegenwart von zweiwertigen Kationen lagert sich das zunächst adsorbierte S-Schicht-Protein in ein zusammenhängendes kristallines S-Schicht-Gitter um. Die Suspension wird in der Folge neuerlich zentrifugiert, und einmal mit 20 ml einer 10 mM CaCl₂-Lösung gewaschen. In dem so hergestellte Pellet stehen die auf der S-Schicht immobilisierten Funktionen (z.B. Rezeptoren wie Antikörper) in Form einer Suspension in hoher Konzentration zur Verfügung.

Für die Untersuchung im Mikroskop wurden 1 - 2 µl des Pellets in Freon 22 (mit flüssigem Stickstoff gekühlt) eingefroren, und einer Gefrierätzung unterzogen. Zur Untersuchung der Ausbildung des kristallinen Gitters wurde das Präparat im Transmissionselektronenmikroskop ausgewertet. Es konnte eine vollständige Beschichtung der Partikel mit S-Schicht-Protein, das in ein schräges Gitter assemblierte, festgestellt werden.

## Patentansprüche

1. Verfahren zum Erzeugen einer Schicht funktioneller Moleküle auf ein er Trägeroberfläche unter Verwendung von Zelloberftächenschicht-Proteinen (S-Schicht-Proteinen) als Träger der funktionellen Moleküle, bei welchem eine S-Schicht-Proteine in Form von Monomeren oder Oligomeren enthaltende Lösung mit der Trägeroberfläche in Kontakt gebracht wird, eine Lage von S-Schicht-Proteinen auf der Trägeroberfläche abgeschieden wird und in der so gebildeten. Lage eine zweidimensionale kristalline Struktur ausgebildet wird,
**dadurch gekennzeichnet, dass**
zum Abscheiden der S-Schicht-Proteine und zum Ausbilden der kristallinen Struktur verschiedene elektrochemische Bedingungen an der elektrochemischen Grenzschicht auf der Trägeroberfläche eingestellt werden, wobei nach der Abscheidung der S-Schicht-Proteine die Kristallisation und Fixierung der S-Schicht-Proteine durch Einstellung des elektrischen Potenzials des Substrats gegenüber einer Referenzelektrode elektrochemisch kontrolliert werden.

2. Verfahren nach Anspruch 1, **dadurch gekennzeichnet, dass** das Abscheiden der S-Schicht-Proteine und das Ausbilden der kristallinen Struktur potentiostatisch kontrolliert erfolgen.

3. Verfahren nach Anspruch 2, **dadurch gekennzeichnet, dass** das Abscheiden der S-Schicht-Proteine und das Ausbilden der kristallinen Struktur voneinander zeitlich getrennt und unter verschiedenen elektrochemischen Bedingungen der Lösung und/oder des Substrats potentiostatisch kontrolliert erfolgen.

4. Verfahren nach Anspruch 2 oder 3, **dadurch gekennzeichnet, dass** zur Änderung der elektrochemischen Bedingungen zwischen Abscheiden und Kristallisation zumindest ein chemischer Parameter der Lösung variiert wird.

5. Verfahren nach Anspruch 2 bis 4, **dadurch gekennzeichnet, dass** zur Änderung der elektrochemischen Bedingungen zwischen Abscheiden und Kristallisation zumindest ein elektrochemischer Parameter des Substrats variiert wird.

6. Verfahren nach einem der Ansprüche 1 bis 5, **dadurch gekennzeichnet, dass** bei dem Abscheiden der S-Schicht-Proteine und/oder dem Ausbilden der kristallinen Struktur eine Konformationsänderung der Proteine eintritt, insbesondere eine Denaturierung bzw. Renaturierung.

7. Verfahren nach einem der Ansprüche 1 bis 6, **dadurch gekennzeichnet, dass** das Abscheiden der S-Schicht-Proteine und/oder das Ausbilden der kristallinen Struktur durch einen zeitlich variierten Potenzialverlauf gesteuert wird.

8. Verfahren nach einem der Ansprüche 1 bis 7, **dadurch gekennzeichnet, dass** das Abscheiden der S-Schicht-Proteine auf das Substrat in einer ersten Lösung und das Ausbilden der kristallinen Struktur in einer zweiten Lösung durchgeführt wird.

9. Verfahren nach Anspruch 8, **dadurch gekennzeichnet, dass** dem Substrat vor dem Eintauchen in die erste Lösung eine Nettoladung elektrostatisch aufgeprägt wird, welche bei dem Durchgang durch die Lösungen erhalten bleibt.

10. Verfahren nach Anspruch 8 oder 9, **dadurch gekennzeichnet, dass** dem Substrat in der ersten Lösung elektrochemisch eine Nettoladung aufgeprägt wird, welche bei dem Wechsel der Lösungen erhalten bleibt.

11. Verfahren nach einem der Ansprüche 8 bis 10, **dadurch gekennzeichnet, dass** der Wechsel der Lösungen durch Transport der Trägeroberfläche von einem ersten Lösungsbad (für das Abscheiden) zu einem zweiten (für die Kristallisation) geschieht.

12. Verfahren nach einem der Ansprüche 1 bis 11, **dadurch gekennzeichnet dass** funktionelle Moleküle an S-Schicht-Proteine noch vor der Abscheidung der S-Schicht-Proteine gebunden werden und sodann die funktionellen Moleküle auf das Substrat zugleich mit dem Abscheiden der S-Schicht-Proteine abgelagert werden.

13. Verfahren nach einem der Ansprüche 1 bis 12, **dadurch gekennzeichnet, dass** nach der Ausbildung der kristallinen Struktur in der S-Schicht-Lage funktionelle Moleküle auf das Substrat an durch die kristalline Struktur definierte Positionen abgelagert werden.

14. Verfahren nach einem der Ansprüche 1 bis 13, **dadurch gekennzeichnet, dass** nach der Ausbildung der kristallinen Struktur in der S-Schicht-Lage elektrochemisch Nanopartikel auf das Substrat an durch die kristalline Struktur definierte Positionen abgelagert werden.

## Claims

1. A method for producing a layer of functional molecules on a carrier surface using cell surface layer proteins (S-layer proteins) as carriers of the functional molecules, in which a solution comprising S-layer proteins in the form of monomers or oligomers is brought into contact with the carrier surface, a coating of S-layer proteins is deposited on the carrier surface and a two-dimensional crystalline structure is formed in the coating thus formed,
**characterized in that**
for the deposition of the S-layer proteins and for the formation of the crystalline structure different electrochemical conditions are set at the electrochemical interface on the carrier surface, and following the deposition of the S-layer proteins the crystallization and fixation of the S-layer proteins is controlled electrochemically by adjustment of the electrical potential of the substrate against a reference electrode.

2. The method as defined in claim 1, **characterized in that** the deposition of the S-layer proteins and the formation of the crystalline structure are carried out in a potentiostatically controlled manner.

3. The method as defined in claim 2, **characterized in that** the deposition of the S-layer proteins and the formation of the crystalline structure are carried out separately with a time lapse therebetween in a potentiostatically controlled manner and under different electrochemical conditions of the solution and/or of the substrate.

4. The method as defined in claim 2 or claim 3, **characterized in that** to change the electrochemical conditions between deposition and crystallization at least one chemical parameter of the solution is varied.

5. The method as defined in any one of claims 2 to 4, **characterized in that** to change the electrochemical conditions between deposition and crystallization at least one electrochemical parameter of the substrate is varied.

6. The method as defined in any one of claims 1 to 5, **characterized in that** during the deposition of the S-layer proteins and/ or the formation of the crystalline structure a conformational change of the proteins occurs, in particular a denaturation or renaturation thereof.

7. The method as defined in any one of claims 1 to 6, **characterized in that** the deposition of the S-layer proteins and/ or the formation of the crystalline structure is controlled by a temporally varied potential profile.

8. The method as defined in any one of claims 1 to 7, **characterized in that** the deposition of the S-layer proteins on the substrate is carried out in a first solution and the formation of the crystalline structure is carried out in a second solution.

9. The method as defined in claim 8, **characterized in that** prior to immersion of the substrate in the first solution a net charge is impressed electrostatically on the substrate, which charge is maintained during passage through the solutions.

10. The method as defined in claim 8 or 9, **characterized in that** in the first solution a net charge is electrochemically impressed on the substrate, which charge is maintained during the change of solutions.

11. The method as defined in any one of claims 8 to 10, **characterized in that** the change of solutions is effected by causing the carrier surface to be transported from a first solution bath (for deposition) to a second one (for crystallization).

12. The method as defined in any one of claims 1 to 11, **characterized in that** functional molecules are bound to S-layer proteins prior to the deposition of the S-layer proteins, whereupon the functional molecules are deposited on the substrate at the same time as the deposition of the S-layer proteins.

13. The method as defined in any one of claims 1 to 12, **characterized in that**, following the formation of the crystalline structure in the S-layer coating, functional molecules are deposited on the substrate at sites defined by the crystalline structure.

14. The method as defined in any one of claims 1 to 13, **characterized in that**, following the formation of the crystalline structure in the S-layer coating, nanoparticles are deposited electrochemically on the substrate at sites defined by the crystalline structure.

## Revendications

1. Procédé pour produire une couche de molécules fonctionnelles sur une surface porteuse en utilisant des protéines de la couche de surface cellulaire (protéines de la couche S) comme support des molécules fonctionnelles, dans lequel une protéine de la couche S sous forme de solution contenant des monomères ou des oligomères est mise en contact avec la surface porteuse, une couche de protéines de la couche S est déposée sur la surface porteuse et une structure cristalline bidimensionnelle est formée dans la couche ainsi formée,
**caractérisé en ce**
**qu'**en vue du dépôt des protéines de la couche S et en vue de la formation de la structure cristalline, différentes conditions électrochimiques sont réglées sur la couche limite électrochimique sur la surface porteuse, la cristallisation et la fixation des protéines de la couche S étant contrôlées électrochimiquement après le dépôt des protéines de la couche S par le réglage du potentiel électrique du substrat par rapport à une électrode de référence.

2. Procédé selon la revendication 1, **caractérisé en ce que** le dépôt des protéines de la couche S et la formation de la structure cristalline sont effectuées sous contrôle potentiostatique.

3. Procédé selon la revendication 2, **caractérisé en ce que** le dépôt des protéines de la couche S et la formation de la structure cristalline sont effectuées séparément chronologiquement et sous contrôle potentiostatique dans diverses conditions électrochimiques de la solution et/ou du substrat.

4. Procédé selon la revendication 2 ou 3, **caractérisé en ce que** qu'en vue de la modification des conditions électrochimiques entre le dépôt et la cristallisation, au moins un paramètre chimique de la solution est varié.

5. Procédé selon les revendications 2 à 4, **caractérisé en ce qu'**en vue de la modification des conditions électrochimiques entre le dépôt et la cristallisation, au moins un paramètre électrochimique du substrat est varié.

6. Procédé selon l'une quelconque des revendications 1 à 5, **caractérisé en ce que** lors du dépôt des protéines de la couche S et/ou de la formation de la structure cristalline, il apparaît une modification conformationnelle des protéines, en particulier une dénaturation et/ou une renaturation.

7. Procédé selon l'une quelconque des revendications 1 à 6, **caractérisé en ce que** le dépôt des protéines de la couche S et/ou la formation de la structure cristalline est commandée par une courbe de potentiel variée chronologiquement.

8. Procédé selon l'une quelconque des revendications 1 à 7, **caractérisé en ce que** le dépôt des protéines de la couche S sur le substrat est effectué dans une première solution et **en ce que** la formation de la structure cristalline est effectuée dans une seconde solution.

9. Procédé selon la revendication 8, **caractérisé en ce qu'**une charge nette est marquée électrostatiquement sur le substrat avant l'immersion dans la première solution, laquelle charge reste constante lors du passage par les solutions.

10. Procédé selon la revendication 8 ou 9, **caractérisé en ce qu'**une charge nette est marquée électrochimiquement sur le substrat dans la première solution, laquelle charge reste constante lors du changement des solutions.

11. Procédé selon l'une quelconque des revendications 8 à 10, **caractérisé en ce que** le changement des solutions est effectué par le transport de la surface porteuse d'un premier bain de solution (pour le dépôt) à un second (pour la cristallisation).

12. Procédé selon l'une quelconque des revendications 1 à 11, **caractérisé en ce que** des molécules fonctionnelles sont encore liées, avant le dépôt des protéines de la couche S, à des protéines de la couche S, et **en ce que**, après, les molécules fonctionnelles sont déposées sur le substrat en même temps que les protéines de la couche S sont déposées.

13. Procédé selon l'une quelconque des revendications 1 à 12, **caractérisé en ce qu'**après la formation de la structure cristalline dans la couche de la couche S, des molécules fonctionnelles sont déposées sur le substrat à des positions définies par la structure cristalline.

14. Procédé selon l'une quelconque des revendications 1 à 13, **caractérisé en ce qu'**après la formation de la structure cristalline dans la couche de la couche S, des nanoparticules sont déposées électrochimiquement sur le substrat à des positions définies par la structure cristalline.
